# EUROPEAN PATENT APPLICATION

(11) **EP 0 867 182 A2**
(43) Date of publication of application: **30.09.1998**
(21) Application number: 98302214.6
(22) Date of filing: 24.03.1998
(51) Int. Cl.: A61K 31/435, A61K 31/445

(54) **The use of certain NK-1 receptor antagonists for the manufacture of a medicament for treating emesis**

(30) Priority: 28.03.1997 US 42038 P
(71) Applicant: PFIZER INC., New York, N.Y. 10017 (US)
(72) Inventor: Nagahisa, Atsushi, Mizuho, Nagoya, Aichi 467 (JP); Tsuchiya, Megumi, Aichi 470-2216 (JP); Silberman, Sandra Leta, Waterford, Connecticut 06385-3511 (US)
(74) Representative: Wood, David John

(57) **Abstract**

The use of a compound of the formula: -
(2S,3 S)-3-(2-methoxy-5-trifluoromethoxybenzyl)amino-2-phenylpiperidine;
(2S,3 S)-N-(5-tert-butyl-2-methoxyphenyl)methyl-2-diphenylmethyl-1-azabicyclo-[2.2.2]octan-3-amine; or
(2S,3 S)-N-(5-isopropyl-2-methoxyphenyl)methyl-2-diphenylmethyl-1-azabicyclo-[2.2.2]octan-3-amine;
or of a pharmaceutically acceptable salt of one of the foregoing compounds, for the manufacture of a medicament for preventing or treating delayed emesis in a mammal.

## Description

The present invention relates to the use of certain NK-1 receptor antagonists (eg., substance P receptor antagonists) to treat delayed emesis.

"Delayed emesis" refers to nausea or vomiting that occurs during the period beginning twenty four hours after the administration of, or expose to an emetic agent and the end of the fifth day following such administration or exposure. Examples of emetic agents are drugs such as cancer chemotherapeutic agents (e.g., cyclophosphamide, carmustine, lomustine and chlorambucil), cytotoxic antibiotics (e.g., dactinomycin, doxorubicin, mitomycin-C and bleomycin), opioid analgesics (e.g., morphine), anti-metabolites (e.g., cytarabine, methotrexate and 5-fluorouracil), vinca alkaloids (e.g., etoposide, vinblastine and vincristine), and other drugs such as cisplatin, ipecac, dacarbazine, procarbazine and hydroxyurea. Emesis may also be induced by radiation sickness, radiation therapy, poisons, toxins such as those caused by metabolic disorders or by infection (e.g., gastritis), pregnancy, vestibular disorders such as motion sickness, postoperative sickness, gastrointestinal obstruction, reduced gastrointestinal motility, visceral pain (e.g., myocardial infarction or peritonitis), migraine, increased intercranial pressure or decreased intercranial pressure (e.g., altitude sickness).

Delayed emesis is a distinct syndrome, the pathophysiology of which is unknown and may be different from that causing acute emesis. Kris et al., Cancer, 70:1012-1016, 1992. Delayed emesis is the major vomiting problem incurred by patients receiving cisplatin chemotherapy. Kris et al., in the foregoing reference, report that, despite excellent control of vomiting during the initial 24 hours of chemotherapy with combination antiemetics, most patients who received cisplatin at dose of 120 mg/m² experienced delayed emesis 24-120 hours after chemotherapy.

The results of clinical studies aimed at determining the incidence, course and severity of delayed emesis following administration of cisplatin, and the effect of treating delayed emesis with specific antiemetic agents or combinations of such agents, are discussed in the following references: Kris et al., Journal of Clinical Oncology, 3:1379-84, 1985; Grumberg et al, J. Nat'I. Cancer Inst., 80: 864-868, 1988; Kris et al., Journal of Clinical Oncology, 7: 108-114, 1989; and Kris et al., Cancer, 70: 1012-1016, 1992.

The use of NK-1 receptor antagonist for the treatment of emesis is referred to in United States Patent 5,393,762, which issued on February 28,1995, United States Patent Application 08/529,891, which was filed on September 18, 1995, European Patent Application EP 533,280A1, which was published on March 24, 1993, and European Patent Application EP 615,751A1, which was published on September 21, 1994. The use of NK-1 receptor antagonists in combination with 5HT₃ receptor antagonists for the treatment of emesis is referred to in United States Patent 5,576,317, which issued on November 19, 1996.

### Summary Of The Invention

The present invention relates to a method of treating or preventing delayed emesis in a mammal, including a human, comprising administering to such mammal a therapeutically effective amount of an NK-1 receptor antagonist selected from the compounds described below.

A "therapeutically effective amount", as used herein, means an amount effective in preventing or decreasing the severiety or the number of occurrences of the nausea and vomiting associated with delayed emesis, as defined above.

"Treating delayed emesis", as used herein, means decreasing the severity or the number of occurrences of the nausea and vomiting associated with delayed emesis, as defined above.

More specifically, this invention relates to a method of preventing or treating delayed emesis in a mammal, including a human, comprising administering to such mammal a therapeutically effective amount of (2S,3S)-N-(5-isopropyl-2-methoxyphenyl)methyl-2-diphenylmethyl-1-azabicyclo[2.2.2]octan-3-amine, or a pharmaceutically acceptable salt of such compound.

This invention also relates to a method of preventing or treating delayed emesis in a mammal, including a human, comprising administering to such mammal a therapeutically effective amount of (2S,3S)-N-(5-tert-butyl-2-methoxyphenyl)methyl-2-diphenylmethyl-1-azabicyclo[2.2.2]octan-3-amine or a pharmaceutically acceptable salt of such compound.

This invention also relates to a method of preventing or treating delayed emesis in a mammal, including a human, comprising administering to such mammal a therapeutically effective amount of (2S,3S)-3-(2-methoxy-5-trifluoromethoxybenzyl)amino-2-phenylpiperidine or a pharmaceutically effective salt of such compound.

A preferred embodiment of this invention relates to a method of preventing or treating delayed emesis in a human, wherein said delayed emesis is caused by a chemotherapeutic agent, comprising administering to said human a therapeutically effective amount of (2S, 3S)-N-(5-isopropyl-2-methoxyphenyl)methyl-2-diphenylmethyl-1-azabicyclic[2.2.2]octan-3-amino or a pharmaceutically acceptable salt of such compound.

A more preferred embodiment of the invention relates to the method described immediately above, wherein the chemotherapeutic agent is or contains cisplatin.

Another preferred embodiment of this invention relates to a method of treating delayed emesis in a human wherein said delayed emesis is caused by a chemotherapeutic agent, comprising administering to said human or therapeutically effective amount of (2S, 3S)-N-(5-t-butyl-2-methoxyphenyl)methyl-2-diphenylmethyl-1-azabicyclic[2.2.2]octan-3-amino or a pharmaceutically acceptable salt of such compound.

A more preferred embodiment of the invention relates to the method described immediately above, wherein the chemotherapeutic agent is or contains cisplatin.

### Detailed Description Of The Invention

The compound (2S,3S)-3-(2-methoxy-5-trifluoromethoxybenxyl)amino-2-phenylpiperidine (also referred to herein as "the piperidine derivative") and its pharmaceutically acceptable salts may be prepared as described in World Patent Application WO 93/00331, which was published on January 7, 1993, United States Patent Application 08/167,881, which was filed on December 14, 1993, and in United States Patent 5,232,929, which issued on August 3, 1993. The compounds (2S,3S)-N-(5-tert-butyl-2-methoxyphenyl)methyl-2-diphenylmethyl-1-azabicyclo[2.2.2]octan-3-amine and (2S,3S)-N-(5-isopropyl-2-methoxyphenyl)methyl-2-diphenylmethyl-1-azabicyclic[2.2.2]octan-3-amino (also referred to herein, collectively, as "the quinuclidine derivatives") and their pharmaceutically acceptable salts may be prepared as described in United States Patent 5,162,339, which issued on November 11, 1992, and United Patent Application 08/211,120, which was filed on May 23, 1994. All of the patents and patents applications mentioned above are incorporated herein by reference in their entireties.

The quinuclidine derivatives employed in the methods of this invention may be prepared by subjecting a compound of the formula 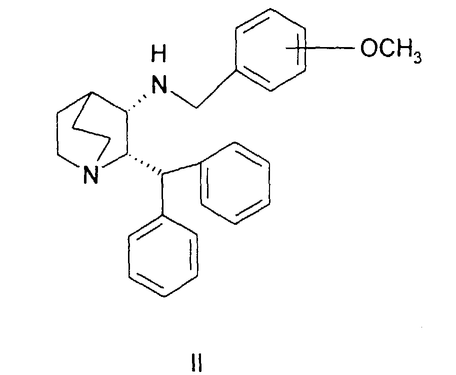 to hydrolytic removal of the methoxybenzyl group to produce the corresponding compound of the formula 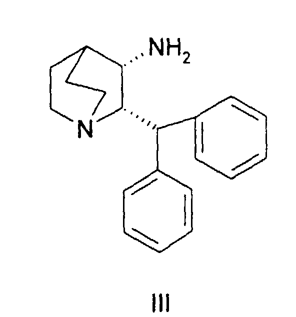 and then reacting the compound of formula III so formed with an aldehyde of the formula 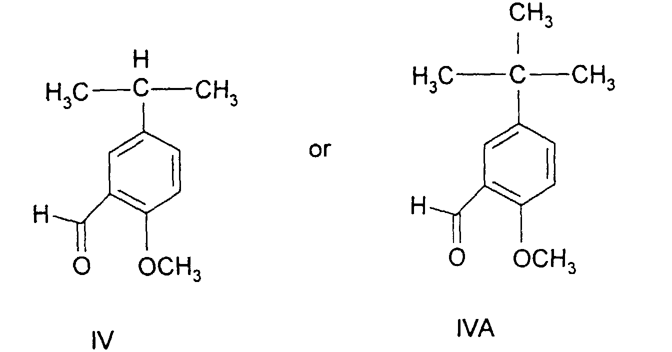 in the presence of a reducing agent.

Hydrolytic removal of the methoxybenzyl group is generally carried out using a strong mineral acid such as hydrochloric, hydrobromic or hydroiodic acid, at a temperature from about room temperature to about the reflux temperature of the acid. Preferably, the reaction is conducted in hydrobromic acid at the reflux temperature. This reaction is usually carried out for a period of about 2 hours.

Altematively, the hydrolytic removal of the methoxybenzyl group in the above procedure may be replaced by hydrogenolytic removal of such group. Hydrogenolytic removal is generally accomplished using hydrogen in the presence of a metal containing catalyst such as platinum or palladium. This reaction is usually conducted in a reaction inert solvent such as acetic acid or a lower alcohol, at a temperature from about 0°C to about 50°C. The methoxybenzyl group may also be removed, alternatively, by treating the compound of formula II with a dissolving metal such as lithium or sodium in ammonia at a temperature from about -30°C to about 78°C, or with a formate salt in the presence of palladium or with cyclohexane in the presence of palladium.

Preferably, the methoxybenzyl group is removed by treating the compound of formula II with hydrogen in the presence of palladium hydroxide on carbon in methanol containing hydrochloric acid at a temperature of about 25°C.

The resulting compound of formula III may be converted into the desired product by reaction with the appropriate aldehyde of formula IV or IVA in the presence of a reducing agent. The reaction is typically carried out using a reducing agent such as sodium cyanoborohydride, sodium triacetoxyborohydride, sodium borohydride, hydrogen and a metal catalyst, zinc and hydrochloric acid, borane dimethylsulfide or formic acid at a temperature from about -60°C to about 50°C. Suitable reaction inert solvents for this reaction include lower alcohols (e.g., methanol, ethanol and isopropanol), acetic acid, methylene chloride and tetrahydrofuran (THF). Preferably, the solvent is methylene chloride, the temperature is about 25°C, and the reducing agent is sodium triacetoxyborohydride.

Alternatively, the reaction of a compound of the formula III with a compound of the formula IV or IVA may be carried out in the presence of a drying agent or using an apparatus designed to remove azeotropically the water generated, to produce an imine of the formula 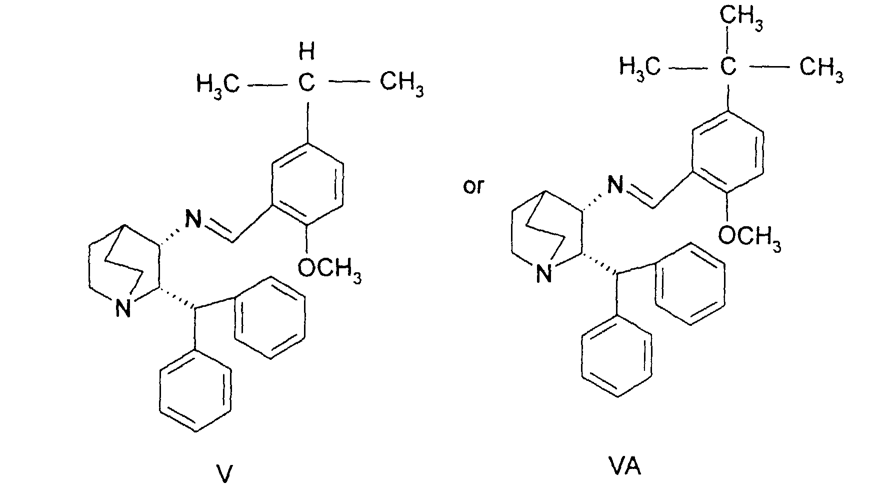 which is then reacted with a reducing agent as described above, preferably with sodium triacetoxyborohydride at about room temperature. The preparation of the imine is generally carried out in a reaction inert solvent such as benzene, xylene or toluene, preferably toluene, at a temperature from about 25°C to about 110°C, preferably at about the reflux temperature of the solvent. Suitable drying agents/solvent systems include titanium tetrachloride/dichloromethane, titanium isopropoxide/dichloromethane and molecular sieves/THF. Titanium tetrachloride/dichloromethane is preferred.

Compounds of the formula III may also be converted into the desired quinuclidine derivative by reaction with the appropriate compound of the formula 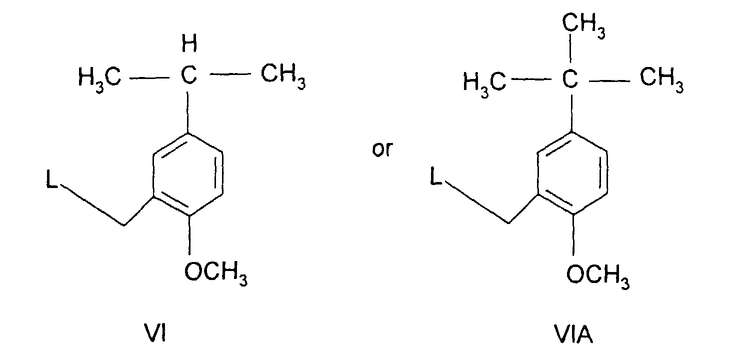 wherein L is a leaving group (e.g., chloro, bromo, iodo or mesylate). This reaction is generally carried out in a reaction inert solvent such as dichloromethane or THF, preferably dichloromethane, at a temperature from about 0°C to about 60°C, preferably at about 25°C.

Compounds of the formula III may also be converted into the desired quomic;odome derivative by reacting them with the appropriate compound of the formula 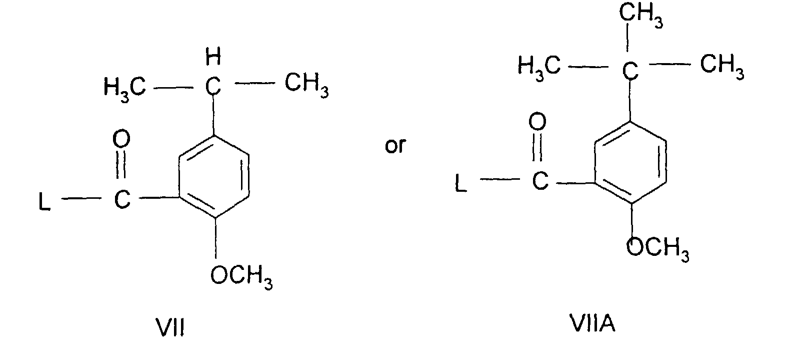 wherein L is defined as above or is imidazole, and then reducing the resulting amide. This reaction is typically carried out in an inert solvent such as THF or dichloromethane at a temperature from about -20°C to about 60°C, preferably in dichloromethane at about 0°C. Reduction of the resulting amide is accomplished by treatment with a reducing agent such as borane dimethylsulfide complex, lithium aluminum hydride or diisobutylaluminum hydride in an inert solvent such as ethyl ether or THF. The reaction temperature may range from about 0°C to about the reflux temperature of the solvent. Preferably, the reduction is accomplished using borane dimethylsulfide complex in THF at about 60°C.

The three NK-1 receptor antogonists employed in the methods of this invention are basic in nature and are capable of forming a wide variety of different salts with various inorganic and organic acids. Although such salts must be pharmaceutically acceptable for administration to animals, it is often desirable in practice to initially isolate the compound of interest from the reaction mixture as a pharmaceutically unacceptable salt and then simply convert the latter back to the free base compound by treatment with an alkaline reagent and subsequently convert the latter free base to a pharmaceutically acceptable acid addition salt. The acid addition salts of the base compounds of this invention are readily prepared by treating the base compound with a substantially equivalent amount of the chosen mineral or organic acid in an aqueous solvent medium or in a suitable organic solvent, such as methanol or ethanol. Upon careful evaporation of the solvent, the desired solid salt is readily obtained.

Generally, in carrying out the methods of this invention, the NK-1 receptor antagonist will be administered to an adult human in an amount ranging from about 0.10 to about 22 mg per kg body weight of the subject being treated per day, in single or divided doses, preferably from about 0.36 to about 4.3 mg/kg per day. Variations may nevertheless occur depending upon the species of the subject being treated and its individual response to said medicament, as well as on the type of pharmaceutical formulation chosen and the time period and interval at which such administration is carried out In some instances, dosage levels below the lower limit of the aforesaid range may be more than adequate, while in other cases still larger doses may be employed without causing any harmful side effect, provided that such larger doses are first divided into several small doses for administration throughout the day.

The NK-1 receptor antagonists and their pharmaceutically acceptable salts that are employed in the methods of this invention are hereinafter also referred to, collectively, as the "therapeutic agents". The therapeutic agents can be administered via either the oral or parenteral route.

The therapeutic agents may be administered alone or in combination with pharmaceutically acceptable carriers or diluents by either of the routes previously indicated, and such administration may be carried out in single or multiple doses. More particularly, the novel therapeutic agents of this invention can be administered in a wide variety of different dosage forms, i.e., they may be combined with various pharmaceutically acceptable inert carriers in the form of tablets, capsules, lozenges, troches, hard candies, suppositories, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the therapeutic compounds of this invention are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a therapeutic agent in either sesame or peanut oil or in aqueous propylene glycol may be employed. The aqueous solutions should be suitably buffered if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

The activity of the therapeutic agents as substance P receptor antagonists may be determined by their ability to inhibit the binding of substance P at its receptor sites in bovine caudate tissue, employing radioactive ligands to visualize the tachykinin receptors by means of autoradiography. The substance P antagonizing activity of the herein described compounds may be evaluated by using the standard assay procedure described by M. A. Cascieri et al., as reported in the Journal of Biological Chemistry, Vol. 258, p. 5158 (1983). This method essentially involves determining the concentration of the individual compound required to reduce by 50% the amount of radiolabelled substance P ligands at their receptor sites in said isolated cow tissues, thereby affording characteristic IC₅₀ values for each compound tested.

In this procedure, bovine caudate tissue is removed from a -70°C freezer and homogenized in 50 volumes (w./v.) of an ice-cold 50 mM Tris (i.e., trimethamine which is 2-amino-2-hydroxymethyl-1,3-propanediol) hydrochloride buffer having a pH of 7.7. The homogenate is centrifuged at 30,000 x G for a period of 20 minutes. The pellet is resuspended in 50 volumes of Tris buffer, rehomogenized and then recentrifuged at 30,000 x G for another twenty-minute period. The pellet is then resuspended in 40 volumes of ice-cold 50 mM Tris buffer (pH 7.7) containing 2 mM of calcium chloride, 2 mM of magnesium chloride, 4 µg/ml of bacitracin, 4µg/ml of leupeptin, 2µg of chymostatin and 200 µg/ml of bovine serum albumin. This step completes the production of the tissue preparation.

The radioligand binding procedure is then carried out in the following manner, viz., by initiating the reaction via the addition of 100 µl of the test compound made up to a concentration of 1 µM, followed by the addition of 100 µl of radioactive ligand made up to a final concentration 0.5 mM and then finally by the addition of 800 µl of the tissue preparation produced as described above. The final volume is thus 1.0 ml, and the reaction mixture is next vortexed and incubated at room temperature (ca. 20°C) for a period of 20 minutes. The tubes are then filtered using a cell harvester, and the glass fiber filters (Whatman GF/B) are washed four times with 50 mM of Tris buffer (pH 7.7), with the filters having previously been presoaked for a period of two hours prior to the filtering procedure. Radioactivity is then determined in a Beta counter at 53% counting efficiency, and the IC₅₀ values are calculated by using standard statistical methods.

## Claims

1. The use of a compound of the formula:-
(2S,3S)-3-(2-methoxy-5-trifluoromethoxybenzyl)amino-2-phenylpiperidine;
(2S,3S)-N-(5-tert-butyl-2-methoxyphenyl)methyl-2-diphenylmethyl-1-azabicyclo-[2.2.2]octan-3-amine; or
(2S,3S)-N-(5-isopropyl-2-methoxyphenyl)methyl-2-diphenylmethyl-1-azabicyclo-2.2.2]octan-3-amine;
or of a pharmaceutically acceptable salt of one of the foregoing compounds, for the manufacture of a medicament for preventing or treating delayed emesis in a mammal.

2. A use according to claim 1, wherein the compound is (2S,3S)-N-(5-isopropyl-2-methoxyphenyl)methyl-2-diphenylmethyl-1-azabicyclo[2,2.2.]octan-3-amine, or a pharmaceutically acceptable salt thereof.

3. The use of (2S,3S)-N-(5-t-butyl-2-methoxyphenyl)methyl-2-diphenylmethyl-1-azabicyclo[2.2.2.]octan-3-amine or (2S,3S)-N-(5-isopropyl-2-methoxyphenyl)methyl-2-diphenylmethyl-1-azabicyclo[2.2.2.]octan-3-amine, or of a pharmaceutically acceptable salt of either of said compounds, for the manufacture of a medicament for preventing or treating delayed emesis in a human caused by an emetogen that is a chemotherapeutic agent.

4. A use according to claim 3, wherein the chemotherapeutic agent is or contains cisplatin.
